# EUROPEAN PATENT SPECIFICATION

(11) **EP 4 634 157 B1**
(45) Date of publication and mention of the grant of the patent: **17.06.2026**
(21) Application number: 23828811.2
(22) Date of filing: 12.12.2023
(51) Int. Cl.: C07D 209/48, C08F 120/36, C09J 175/04, C09J 4/00

(54) **CYCLIC IMIDES AND ADHESIVE COMPOSITIONS INCLUDING THEM**
ZYKLISCHE IMIDE UND UND KLEBSTOFFZUSAMMENSETZUNGEN DIESE ENTHALTEND
IMIDES CYCLIQUES ET COMPOSIITONS ADHESIVES LES COMPRENANT

(30) Priority: 15.12.2022 US 202263432939 P
(43) Date of publication of application: 22.10.2025
(73) Proprietor: 3M Innovative Properties Company, Saint Paul, Minnesota 55133-3427 (US)
(72) Inventor: OSTLUND, Anthony J., Saint Paul, Minnesota 55133-3427 (US); KRYGER, Matthew J., Saint Paul, Minnesota 55133-3427 (US)
(74) Representative: Bergen, Katja
(86) International application number: PCT/IB2023/062560
(87) International publication number: WO 2024/127252

(56) References cited:
- WO-A1-2022/034521

## Description

### Cross-Reference to Related Application

This application claims priority to U.S. Provisional Application No. 63/432,939, filed December 15, 2022.

### Background

Structural adhesives are known to be useful for bonding one substrate to another. Structural adhesives are attractive alternatives to mechanical joining methods, such as riveting or spot welding, because structural adhesives distribute load stresses over larger areas rather than concentrating such stresses at a few points.

Int. Pat. Appl. Pub. No. WO 2022/034521 (Ostlund et al.) discloses (meth)acrylate structural adhesive compositions including a cyclic imide-containing monomer. The compositions cure by means of a free-radical initiator system at room temperature, and the resulting structural adhesives are reported to bond well to glass.

### Summary

The present disclosure provides a compound useful, for example, for improving the adhesion between substrates when used, for example, in a structural adhesive composition.

In one aspect, the present disclosure provides a compound represented by formula: 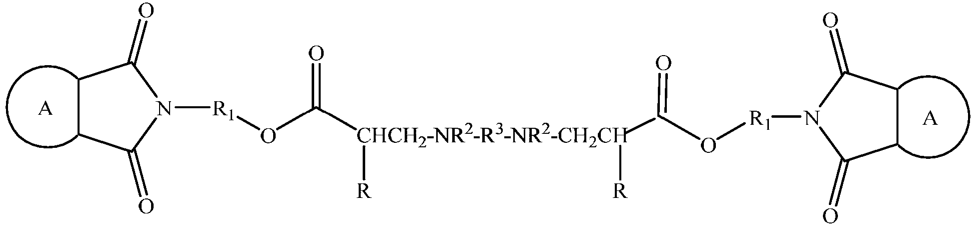 In this formula, A represents a saturated, unsaturated, or aromatic ring; each R is independently hydrogen or methyl; each R¹ is independently alkylene optionally interrupted by one or more -O- groups; each R³ is independently alkylene, arylene, or alkylene optionally interrupted or terminated by arylene, optionally interrupted by one or more -O- groups or one to three -NR²- groups, and optionally terminated by -N(R²)₂; and each R² is independently hydrogen, alkyl, aryl, arylalkylenyl, or

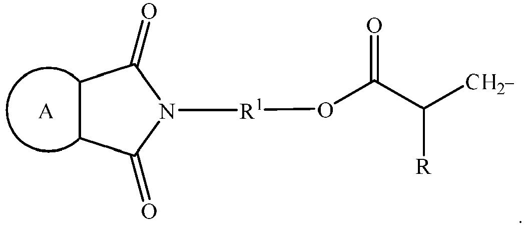

In another aspect, the present disclosure provides an adhesive composition that includes the compound.

In another aspect, the present disclosure provides the use of the compound as an adhesion promoter.

In another aspect, the present disclosure provides a method of bonding a first substrate and a second substrate. The method includes applying the adhesive composition to at least a portion of one surface of the first substrate, contacting at least a portion of the adhesive composition with at least a portion of one surface of the second substrate, and allowing the adhesive composition to at least partially cure and form the structural adhesive.

In another aspect, the present disclosure provides a bonded article prepared according to the method. The bonded article includes the structural adhesive bonded to the first substrate and the second substrate.

In this application:
Terms such as "a", "an" and "the" are not intended to refer to only a singular entity but include the general class of which a specific example may be used for illustration. The terms "a", "an", and "the" are used interchangeably with the term "at least one".

The phrase "comprises at least one of" followed by a list refers to comprising any one of the items in the list and any combination of two or more items in the list. The phrase "at least one of" followed by a list refers to any one of the items in the list or any combination of two or more items in the list.

The terms "cure" and "curable" refer to joining polymer chains together by covalent chemical bonds, usually via crosslinking molecules or groups, to form a network polymer. Therefore, in this disclosure the terms "cured" and "crosslinked" may be used interchangeably. A cured or crosslinked polymer is generally characterized by insolubility but may be swellable in the presence of an appropriate solvent.

The term "polymer" refers to a molecule having a structure which includes the multiple repetition of units derived, actually or conceptually, from one or more monomers. The term "monomer" refers to a molecule of low relative molecular mass that can combine with others to form a polymer. The term "polymer" includes homopolymers and copolymers, as well as blends of homopolymers or copolymers that may be formed in a miscible blend. The term "polymer" includes random, block, graft, and star polymers. The term "polymer" encompasses oligomers.

"Alkyl group" and the prefix "alk-" are inclusive of both straight chain and branched chain groups and of cyclic groups. In some embodiments, alkyl groups have up to 30 carbons (in some embodiments, up to 20, 15, 12, 10, 8, 7, 6, or 5 carbons) unless otherwise specified. Cyclic groups can be monocyclic or polycyclic and, in some embodiments, have from 3 to 10 ring carbon atoms. Terminal "alkenyl" groups have at least 3 carbon atoms.

"Alkylene" is the multivalent (e.g., divalent or trivalent) form of the "alkyl" groups defined above.

"Arylalkylene" refers to an "alkylene" moiety to which an aryl group is attached. "Alkylarylene" refers to an "arylene" moiety to which an alkyl group is attached.

The terms "aryl" and "arylene" as used herein include carbocyclic aromatic rings or ring systems, for example, having 1, 2, or 3 rings and optionally containing at least one heteroatom (e.g., O, S, or N) in the ring optionally substituted by up to five substituents including one or more alkyl groups having up to 4 carbon atoms (e.g., methyl or ethyl), alkoxy having up to 4 carbon atoms, halo (i.e., fluoro, chloro, bromo or iodo), hydroxy, cyano, or nitro groups. Examples of aryl groups include phenyl, naphthyl, biphenyl, fluorenyl as well as furyl, thienyl, pyridyl, quinolinyl, isoquinolinyl, indolyl, isoindolyl, triazolyl, pyrrolyl, tetrazolyl, imidazolyl, pyrazolyl, oxazolyl, and thiazolyl.

The phrase "interrupted by arylene", for example, with regard to an alkylene group refers to having part of the alkylene on both sides of the arylene group. For example, -CH₂CH₂-C₆H₅-CH₂-CH₂- is an alkylene group interrupted by a phenylene group. Similarly, -CH₂CH₂-NH-CH₂-CH₂- is an alkylene group interrupted by an -NH- group.

All numerical ranges are inclusive of their endpoints and non-integral values between the endpoints unless otherwise stated (e.g., 1 to 5 includes 1, 1.5, 2, 2.75, 3, 3.80, 4, 5, etc.).

### Detailed Description

The compound of the present disclosure is represented by formula

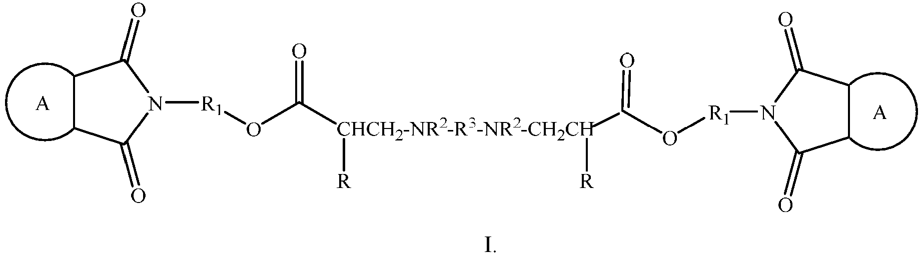

In formula I, each A independently represents a saturated, unsaturated, or aromatic ring. In some embodiments, each A independently represents a 5- to 8-membered ring. In some embodiments, each A independently represents a 5- to 8- (in some embodiments, 5- to 7-) membered, saturated ring. In some embodiments, each A independently represents a 6-membered, saturated ring. In formula I, each R is independently hydrogen or methyl. In some embodiments, each R is hydrogen. In some embodiments, each R is methyl. In formula I, each R¹ is independently alkylene optionally interrupted by one or more -O- groups. In some embodiments, each R¹ is independently alkylene having from 1 to 18 carbon atoms. In some embodiments, each R¹ is independently alkylene (in some embodiments, linear alkylene) having from 2 to 16, 2 to 12, 2 to 8, 2 to 6, 2 to 4, or 2 carbon atoms.

In formula I, R³ is independently alkylene, arylene, or alkylene optionally interrupted or terminated by arylene, optionally interrupted by one or more -O- groups or one to three -NR²- groups, and optionally terminated by -N(R²)₂. When R³ is terminated by -N(R²)₂, R³ is a branched group (e.g., R³ is trivalent). In some embodiments, R³ is a multivalent alkylene group optionally interrupted by one or more -O- groups or up to three -NR²- groups. In some embodiments, R³ is a multivalent alkylene group optionally interrupted by one or more -O- groups. In some of these embodiments, multivalent alkylene group comprises a cyclohexane ring. In some embodiments, R³ is -CH-(CH₃)CH₂-O-CH₂-C₆H₁₀-CH₂-O-CH₂-CH(CH₃)-. In some embodiments, R³ is comprises polyethylene oxide, polypropylene oxide, or a combination thereof, in some embodiments, having a number average molecular weight of up to 4000, 3000, 2000, 1000, or 500 grams per mole as determined by Proton Nuclear Magnetic Resonance Spectroscopy. In some embodiments, R³ is alkylene interrupted by arylene, in some embodiments, -CH₂-C₆H₅-CH₂-.

In formula I, each R² is independently each R² is independently hydrogen, alkyl, aryl, arylalkylenyl, or 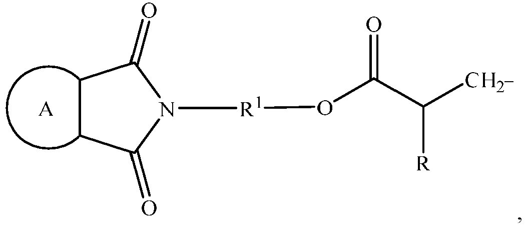 where A, R, and R¹ are defined as in any of the above embodiments. In some embodiments, all R² groups are hydrogen, and the compound comprises two cyclic imide groups. In some embodiments, each R² is 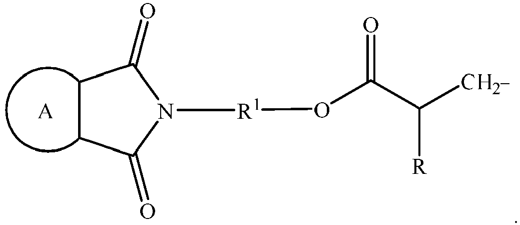 In some of these embodiments, R³ does not include -NR²- groups, and the compound comprises four cyclic imide groups. In some embodiments, each R² is independently hydrogen or 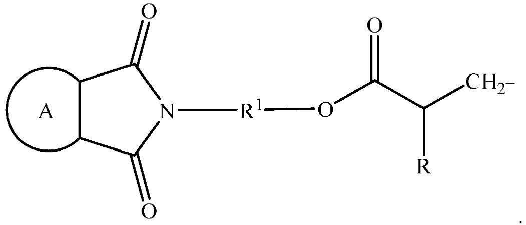 In some embodiments, the compound comprises three cylic imide groups. **In** some of these embodiments, R³ is terminated by -N(R²)₂.

The compound of the present disclosure can be made, for example, by reacting a multi-functional amine with an alpha-beta unsaturated compound including a cyclic amide under conventional Michael addition reaction conditions. Useful multi-functional amines have at least two amino groups and may be aromatic, aliphatic (e.g., linear or cycloaliphatic), or a combination thereof. The amino groups in the multi-functional amine are each independently primary or secondary amino groups. **In** some embodiments, the multi-functional amine includes at least two primary amino groups.

Useful multi-functional amines may be aliphatic amines including at least two amino groups. In some embodiments, the multi-functional amine is a linear or branched alkylene polyamine. Useful alkylene polyamines include ethylene amines (e.g., ethylenediamine, diethylenetriamine, triethylenetetramine, etc.), higher alkylenediamines (e.g., hexamethylenediamine, methylpentamethylenediamine, and trimethylhexanediamine), and polyetheramines (e.g., polyoxyalkylene diamines such as polyoxypropylene diamines of various molecular weights and 4,7,10-trioxa-1,13-tridecane diamine).

The multi-functional amine may be an aromatic polyamine, in which the amino groups are bonded directly to the aromatic ring, or an arylalkylenyl polyamine, in which the amino groups are bonded to alkylene groups that are in turn bonded to the aromatic ring. The multi-functional amine may also contain two or more aromatic rings and at least two amino groups. In any of these embodiments, the aromatic ring can be unsubstituted or substituted by one or more halogens (e.g., fluoro, chloro, bromo, iodo), alkyl groups having 1 to 4 carbon atoms (e.g., methyl or ethyl), or hydroxyalkyl groups having 1 to 4 carbon atoms (e.g., hydroxymethyl). For amines containing two or more aromatic rings, the rings may be directly connected or connected, for example, by a branched or straight-chain alkylene group having 1 to 4 carbon atoms that may optionally be substituted by one or more halogens (e.g., fluoro, chloro, bromo, iodo), an oxygen, a sulfur, or a sulfone group. Examples of multi-functional amine that comprise at least two amino groups and at least one aromatic ring include phenylenediamine (e.g., meta-phenylenediamine or para-phenylenediamine), diethyl toluene diamine (e.g., in any of its isomeric forms), diamino toluene (e.g., 2,3-diaminotoluene and 3,4-diaminotoluene, and methyl-m-phenylenediamine), 1,2-diamino-3,5-dimethylbenzene, 4,5-dimethyl-1,2-phenylenediamine, 2,4,6-trimethyl-m-phenylenediamine, 2,3,5,6-tetramethyl-p-phenylenediamine, aminobenzylamines (e.g., 2-aminobenzylamine and 4-aminobenzylamine), ethylenedianiline, 2,2'-biphenyldiamine, diaminodiphenylmethane, diaminodiphenylsulfone, halogenated substituted pheneylene diamines (e.g., 4-chloro-1,3-diaminobenzene, 4-chloro-1,2-diaminobenzene, and 4-bromo-1,2-diaminobenzene), a xylylenediamine (e.g., ortho-xylylenediamine or meta-xylylenediamine), and 4-(2-aminoethyl)aniline.

The multi-functional amine may comprise at least two amino groups and at least one cycloaliphatic ring. The amino groups may be bonded directly to the cycloaliphatic ring, or the amino groups may be bonded to straight-chain or branched alkylene groups that are in turn bonded to the cycloaliphatic ring. An amine curing agent may also contain two or more cycloaliphatic rings and at least two amino groups. In any of these embodiments, the cycloaliphatic ring can be unsubstituted or substituted by one or more halogens (e.g., fluoro, chloro, bromo, iodo), straight-chain or branched alkyl groups having 1 to 4 carbon atoms (e.g., methyl or ethyl), or hydroxyalkyl groups having 1 to 4 carbon atoms (e.g., hydroxymethyl). In any of these embodiments, the cycloaliphatic ring may be a carbocyclic ring, for example, including no heteroatoms such as sulfur or nitrogen. For amines containing two or more cycloaliphatic rings, the rings may be directly connected or connected, for example, by a branched or straight-chain alkylene group having 1 to 4 carbon atoms that may optionally be substituted by one or more halogens (e.g., fluoro, chloro, bromo, iodo), an oxygen, a sulfur, or a sulfone group. Examples of suitable amine curing agents that comprise at least two amino groups and at least one cycloaliphatic group are the fully or partially hydrogenated products of any of the amine curing agents that comprise at least two amino groups and at least one aromatic ring described above. For example, suitable amine curing agents include diaminocyclohexanes (e.g., 1,2-diaminocyclohexane or 1,4-diaminocyclohexane in their cis- or trans- forms) and 3-aminomethyl-3,5,5-trimethylcyclohexylamine (also called isophorone diamine).

Several multi-functional amines including at least two amino groups and at least one of an aromatic ring or a cycloaliphatic ring are available, for example, from Lonza, Basel, Switzerland, and Amberlite Corporation, Baton Rouge, LA. Other amine curing agents that may be useful include polyetheramines (e.g., polypropylene glycol diamines) available, for example, from Huntsman Chemical, The Woodlands, TX, under the trade designation "JEFFAMINE".

Any of these multi-functional amines can react with, for example, a cyclic imide-containing acrylate or a cyclic imide-containing methacrylate. The cyclic imide-containing acrylate or a cyclic imide-containing methacrylate comprises a ring system that includes one or more rings, in some embodiments, two rings. The ring system may include one or more saturated rings, unsaturated rings, aromatic rings, or a combination thereof. In some embodiments, the cyclic imide-containing acrylate or a cyclic imide-containing methacrylate is represented by formula: 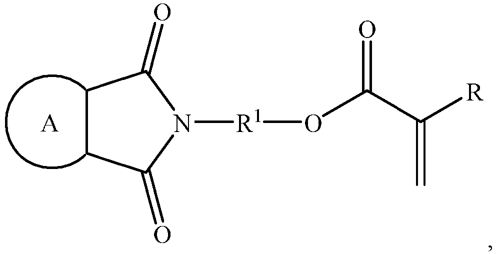
wherein A, R¹, and R are as described above in any of their embodiments. In some embodiments, the cyclic imide-containing acrylate or cyclic imide-containing methacrylate is 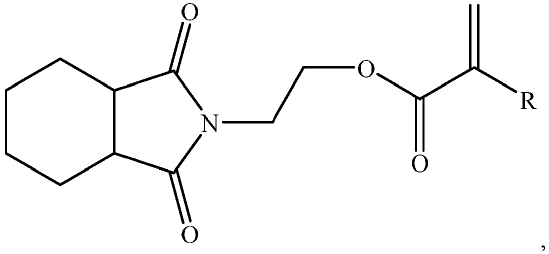
wherein R is hydrogen or methyl (that is, 2-(hexahydrophthalimido)ethyl acrylate or 2-(hexahydrophthalimido)ethyl methacrylate). In some embodiments, the cyclic imide-containing acrylate or cyclic imide-containing methacrylate is 2-(hexahydrophthalimido)ethyl acrylate. 2-(Hexahydrophthalimido)ethyl methacrylate is commercially available, for example, from Miwon North America (Exton, Pa.) under the trade designation "MIRAMER M1089"). 2-(Hexahydrophthalimido)ethyl acrylate is commercially available, for example, from Miwon North America under the trade designation "MIRAMER M1088").

The compound of the present disclosure can be made by combining the multi-functional amine and the cyclic imide-containing acrylate or a cyclic imide-containing methacrylate. The reaction may be carried out neat or in a suitable solvent. Optionally, the reaction mixture can be heated at an elevated temperature, for example, at least 40 °C, at least 50 °C, or at least 60 °C. The upper temperature can be determined by the boiling temperature of the solvent, if used.

The present disclosure provides an adhesive composition comprising the compound described herein. In some embodiments, the adhesive composition is a structural adhesive composition. A "structural adhesive" means an adhesive that binds by irreversible cure. A structure adhesive typically bonds high strength materials (e.g., wood, composites, or metals) with a strength measured as stress at break (peak stress) using the overlap shear test described in the Examples herein, of at least 689 kPa (100 psi), at least 1379 kPa (200 psi), at least 3445 kPa (500 psi), or at least 6890 kPa (1000 psi).

Structural adhesive compositions can be packaged either as two-part products or one-part products. For the two-part products, the two parts can be stored at room temperature. Once the user mixes the two parts, the reaction begins, and the composition starts to form a structural adhesive. After at least partial curing, a crosslinked composition is generally obtained, and if sufficiently cured it may be suitable for use as a structural adhesive to bond two adherends. In such use, the composition is typically sandwiched between the adherends and at least partially cured; for example, for a time sufficient to achieve at least a desired level of bond strength. It is desirable that the adhesive composition exhibit a suitable open time and cure rapidly. The "open time" of a two-part adhesive refers to the amount of time after the two components are mixed that the adhesive remains flowable and capable of bonding to a substrate.

For one-part products that can at least partially cure at room temperature, users can avoid a complicated mixing step, but the product is typically shipped and stored in a freezer before application. One-part products that are cured by actinic radiation (e.g., that do not include a second initiator for curing at room temperature or elevated temperature, may be able to be shipped and stored at room temperature before application, sometimes in the dark. Some one-part structural adhesive compositions require elevated temperatures to cure.

The compound of the present disclosure may be useful in a variety of structural adhesive compositions. These include heat-curable phenolic adhesives, heat-curable epoxy adhesives, heat-curable polyimide adhesives, two-part epoxy adhesives, one-part light curable adhesives (e.g., which may be acrylic adhesives or epoxy adhesives), two-part acrylic adhesives, one-part moisture- or heat-cured polyurethane adhesives, and two-part polyurethane adhesives. In some embodiments, the adhesive composition of the present disclosure is a two-part structural adhesive composition. In some embodiments, the adhesive composition is one part of a two-part structural adhesive composition. In some embodiments, the adhesive composition is a polyurethane adhesive composition. In some embodiments, the adhesive composition is a two-part polyurethane adhesive composition.

Two-part polyurethane adhesive compositions include one part that includes one or more polyisocyanate compounds and a second part that includes one or more polyols. When the two parts are mixed, the polyisocyanates and polyols react to form a cured polyurethane adhesive. A polyurethane adhesive can be formulated to cure at room temperature or upon exposure to certain conditions, such as elevated temperatures. As the adhesive cures, it can form a strong adhesive bond to many types of substrates. The polyurethane may include a backbone of any suitable structural configuration. The backbone may optionally include one or more other backbone linkages (e.g., amide, ester, carbonate ester, epoxy, ether, imide, imine, or urea linkages, or a combination thereof). Moreover, the backbone of the polyurethane polymer may optionally include one or more oligomer or polymer segments (e.g., acrylic, polyamide, polyester, poly(carbonate ester), epoxy, polyether, polyimide, polyimine, or polyurea segments, or a combination thereof). The polyurethane may be linear, substantially linear, or branched.

The polyisocyanate compound can be any compound which contains on average more than one isocyanate moiety. The polyisocyanate compound can be in the form of isocyanate functional prepolymers, monomers, or oligomers having on average greater than 1 isocyanate group, and, in some embodiments, 2 or more isocyanate groups. Isocyanate prepolymers can by any prepolymers prepared by reaction of a polyisocyanate compound with one or more compounds having on average more than one isocyanate reactive functional group (e.g., hydroxyl, amine, thiol, and carboxyl), under conditions such that the prepolymers prepared have on average more than one isocyanate group per molecule. The polyisocyanate compound is typically present in a structural adhesive composition in a sufficient amount to form a cured component when exposed to curing conditions.

Examples of suitable polyisocyanate compounds useful in a structural adhesive composition and useful for preparing isocyanate functional prepolymers include any aliphatic, cycloaliphatic, aromatic/aliphatic, heterocyclic, and aromatic polyisocyanates and combinations thereof. In some embodiments, the polyisocyanates used have an average isocyanate functionality of about 2.0 or greater and an equivalent weight of about 80, 110, or 120 or greater and up to 300, 250, or 200 equivalents per mole. In some embodiments, the isocyanate functionality of the polyisocyanate compound is about 2.2 or greater, about 2.4 or greater, about 4.0 or less, about 3.5 or less, or about 3.0 or less.

Suitable aliphatic diisocyanates include isophoronediisocyanate (i.e.,5-isocyanato-1-isocyanatomethyl-1,3,3-trimethylcyclohexane); 5-isocyanato-1-(2-isocyanatoeth-1-yl)-1,3,3-trimethylcyclohexane; 5-isocyanato-1-(3-isocyanatoprop-1-yl)-1,3,3-trimethylcyclohexane; 5-isocyanato-(4-isocyanatobut-1-yl)-1,3,3-trimethylcyclohexane; 1-isocyanato-2-(3-isocyanatoprop-1-yl)cyclohexane; 1-isocyanato-2-(3-isocyanatoeth-1-yl)cyclohexane; 1-isocyanato-2-(4-isocyanatobut-1-yl)cyclohexane; 1,2-diisocyanatocyclobutane; 1,3-diisocyanatocyclobutane; 1,2-diisocyanatocyclopentane; 1,3-diisocyanatocyclopentane; 1,2-diisocyanatocyclohexane; 1,3-diisocyanatocyclohexane;1,4-diisocyanatocyclohexane; dicyclohexylmethane 2,4'-diisocyanate; trimethylene diisocyanate; tetramethylene diisocyanate; pentamethylenediisocyanate; hexamethylene diisocyanate; ethylethylene diisocyanate; trimethylhexane diisocyanate; heptamethylene diisocyanate; 2-heptyl-3,4-bis(9-isocyanatononyl)-1-pentyl-cyclohexane; 1,2-, 1,4-, and 1,3-bis(isocyanatomethyl)cyclohexane; 1,2-, 1,4-, and1,3-bis(2-isocyanatoeth-1-yl)cyclohexane; 1,3-bis(3-isocyanatoprop-1-yl)cyclohexane; 1,2-, 1,4-or 1,3-bis(4-isocyanatobuty-1-yl)cyclohexane; liquid bis(4-isocyanatocyclohexyl)-methane; and derivatives or mixtures thereof. In some embodiments, the polyisocyanate compound is isophorone diisocyanate, tetramethylxylene diisocyanate, 1,6-hexamethylene diisocyanate and oligomeric or polymeric derivatives thereof, bis(4-isocyanato-cyclohexyl)methane, or trimethyl hexamethylene diisocyanate. In some embodiments, the polyisocyanate compound comprises at least one of isophorone diisocyanate (IPDI) or hexamethylene diisocyanate (HMDI). Suitable aromatic isocyanates include diphenylmethane diisocyanate, toluene diisocyanate, bis(isocyanatoethyl)benzene, alpha, alpha, alpha', alpha'-tetramethylxylene diisocyanate, 1,3-bis(1-isocyanato-1-methylethyl)benzene, bis(isocyanatobutyl)benzene, bis(isocyanatomethyl)naphthalene, bis(isocyanatomethyl)diphenyl ether, bis(isocyanatoethyl)phthalate, mesitylene triisocyanate, 2,5-di(isocyanatomethyl)furan, phenylene diisocyanate, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, naphthalene diisocyanate, methylnaphthalene diisocyanate, biphenyl diisocyanate, ortho-tolidine diisocyanate, 4,4'-diphenylmethane diisocyanate, bis(3-methyl-4-isocyanatophenyl)methane, bis(isocyanatophenyl)ethylene, 3,3'-dimethoxy-biphenyl-4,4'-diisocyanate, triphenylmethane triisocyanate, polymeric 4,4'-diphenylmethane diisocyanate, naphthalene triisocyanate, diphenylmethane-2,4,4'-triisocyanate, 4-methyldiphenylmethane-3,5,2',4',6'-pentaisocyanate, diphenylether diisocyanate, bis(isocyanatophenylether)ethyleneglycol, bis(isocyanatophenylether)-1,3-propyleneglycol, benzophenone diisocyanate, combinations thereof and polymeric derivatives thereof.

Higher functional polyisocyanate compounds include trimeric isocyanurates or biurets of monomeric isocyanates and oligomers and can be aliphatic or aromatic. Examples commercially available isocyanate compounds include trimers of hexamethylene diisocyanate, such as those available from Bayer under the trade designation DESMODUR N3300, DESMODUR N3400, DESMODUR N-100. Suitable oligomeric aromatic polyisocyanates include those available from The Dow Chemical Company under the trademarks PAPI and VORANATE, such as VORANTE M220, PAPI 27 and PAPI 20 polymeric isocyanates.

Suitable polyols for preparing polyurethanes include monomers, oligomers, polymers, and mixtures thereof and include diols, triols, polyols having 4 or more hydroxyl groups, and mixtures thereof. Examples of polyols for use as reactants or as starting materials for oligomer or polymer polyols include ethylene glycol, propylene glycol, 1,3-propanediol, glycerol, diethylene glycol, dipropylene glycol, triethylene glycol, trimethylolpropane, trimethylolethane, tripropyleneglycol, neopentyl glycol, pentaerythritol, 1,4-butanediol, hexyleneglycol, cyclohexanedimethanol, a polyethylene or polypropylene glycol, isopropylidene bis(p-phenylene-oxypropanol-2), and mixtures thereof. Examples of useful polyols include polyether polyols, polyester polyols, poly(alkylene carbonate)polyols, hydroxyl containing polythioethers, polyether-ester polyols, polyureapolyols, polyamide polyols, polycarbonate polyols, saturated or unsaturated polyolefin polyols, and mixtures thereof. In some embodiments, the polyol is a polyether polyol containing one or more alkylene oxide units (e.g., ethylene oxide, propylene oxide, butylene oxide, and mixtures thereof) in the backbone of the polyol. The alkylene oxides can contain straight or branched chain alkylene units. When a mixture of alkylene oxide units is contained in a polyol, the different units can be randomly arranged or arranged in blocks of each alkylene oxide. In some embodiments, the polyol comprises propylene oxide chains with ethylene oxide chains capping the polyol. In some embodiments, the polyol is a mixture of a diol and a triol. In some embodiments, useful polyols have a functionality of about 1.9 or greater, about 1.95 or greater, about 2.0 or greater, about 4.0 or less, about 3.5 or less, or about 3.0 or less. In some embodiments, the polyol has an equivalent weight of at least about 200, 500, or 1,000 or greater and up to about 5,000, 3,000 or 2,500 or less.

Isocyanate prepolymers useful as polyfunctional isocyanates in the adhesive composition of the present disclosure may be prepared by any suitable method, such as bulk polymerization and solution polymerization. The process may be carried out under anhydrous conditions and under an inert atmosphere, such as a nitrogen blanket, to prevent crosslinking of the isocyanate groups by moisture. The reaction can be carried out at a temperature from about 0° C to about 150° C, or from about 25° C to about 90° C, until the residual isocyanate content determined by titration of a sample is close to the desired theoretical value. The reactions to prepare the prepolymer may be carried out in the presence of urethane catalysts, for example stannous salts of carboxylic acids (e.g., stannous octoate, stannous oleate, stannous acetate, and stannous laurate), dialkyltin dicarboxylates (e.g., dibutyltin dilaurate and dibutyltin diacetate), tertiary amines, and tin mercaptides. The amount of catalyst employed is generally from about 0.005 to about 5 parts by weight of the mixture catalyzed. The reaction can be carried out in admixture with a plasticizer. The polyisocyanate and polyol compounds used to make a prepolymer can be any of those described above. The amount of polyisocyanate compound and polyol used to prepare the prepolymer is an amount that gives the desired properties and the desired free isocyanate content and viscosity.

The second part of a two-part polyurethane adhesive composition useful as the adhesive composition (e.g., the structural adhesive composition) of the present disclosure can include one or more curing agents located in the second part such as the polyols described above. Curing agents are compounds that contains greater than one isocyanate-reactive group, in some embodiment, hydroxyl or amine functional groups. The curing agents can be one or more chain extenders, crosslinking agents, polyols, polyamines, or prepolymers prepared as described but having isocyanate-reactive groups as a result of excess equivalents of isocyanate-reactive groups.

The second part of a two-part polyurethane adhesive composition may comprise one or more low molecular weight compounds having two or more isocyanate-reactive groups. Such low molecular weight compounds may be difunctional chain extenders or crosslinkers having greater than two active hydrogen groups per compound. The molecular weight of the low molecular weight compound can be about 120 or less or about 100 or less. The low molecular weight compound can be one or more multifunctional alcohol, multifunctional alkanol amine, one or more adducts of multifunctional alcohol and an alkylene oxide, one or more adducts of a multifunctional alkanol amine and an alkylene oxide, or a mixture thereof. Examples of suitable multifunctional alcohols and multifunctional alkanol amines include ethane diol, propane diol, butane diol, hexane diol, heptane diol, octane diol, glycerine, trimethylol propane, pentaerythritol, neopentyl glycol, ethanol amines (e.g., diethanol amine and triethanol amine) and propanol amines (e.g., di-isopropanol amine and tri-isopropanol amine). In some embodiments, the low molecular weight compound is present in the adhesive composition (e.g., the second part of a two-part polyurethane composition) in an amount of about 2, 3, or percent by weight or greater and about 16, 12, or 10 percent by weight or less, based on the total weight of the second part.

In some embodiments, the second part of a two-part polyurethane adhesive composition comprises a polyoxyalkylene polyamine having 2 or greater amines per polyamine. The polyoxyalkylene polyamine can be any of those described above. In some embodiments, the polyoxyalkylene polyamine is present in the adhesive composition (e.g., the second part of a two-part polyurethane composition) in an amount of about 0.2, 0.3, or 0.5 percent by weight or greater and up to 6, 4, or 2 percent by weight or less, based on the total weight of the second part.

The second part of a two-part polyurethane adhesive composition may comprise one or more catalysts which catalyze the reaction of isocyanate groups with isocyanate-reactive groups. The catalyst may be any of those described above for making polyurethane prepolymers. Organotin compounds or metal alkanoates may be present in an amount of about 60 or 120 parts per million or greater and up to about 1.0, 0.5, or 0.2 percent or less based on the total weight of the second part. Examples of suitable tertiary amine catalysts include dimorpholinodialkyl ether, a di((dialkyl-morpholino)alkyl)ether, bis-(2-dimethylaminoethyl)ether, triethylene diamine, pentamethyldiethylene triamine, N,N-dimethylcyclohexylamine, N,N-dimethyl piperazine 4-methoxyethyl morpholine, N-methylmorpholine, N-ethyl morpholine, diazabicyclo compounds and mixtures thereof. Diazabicyclo compounds are compounds which have diazobicyclo structures, examples of which include diazabicycloalkanes and diazabicyclo alkene salts. Examples of diazabicycloalkanes include diazabicyclooctane, available from Air Products under the trademark and designations, DABCO, DABCO WT, DABCO DC 1, DABCO DC 2, and DABCO DC 21. Examples of diazabicycloalkene salts include diazabicycloundecene in the phenolate, ethylhexoate, oleate and formate salt forms, available from Air Products under the trademark and designations, POLYCAT SA 1, POLYCAT SA 1/10, POLYCAT SA 102 and POLYCAT SA 610. Tertiary amines can be used in an amount, based on the weight of second part of the two-part composition, of about 0.01, 0.05, 0.1, or 0.2 percent by weight or greater and about 2.0, 1.5, or 1.2 percent by weight or less.

Various additives may be included in the adhesive compositions (in some embodiments, structural adhesive composition) of the present disclosure, for example, to alter the characteristics of the adhesive composition. Examples of useful additives include corrosion inhibitors such as some silica gels, thixotropic agents such as fumed silica; pigments (e.g., ferric oxide, brick dust, carbon black, and titanium oxide), reinforcing agents (e.g., silica, magnesium sulfate, calcium sulfate, and beryllium aluminum silicate), clays such as bentonite, and any suitable filler (e.g., glass beads, talc, and calcium metasilicate). Amounts of up to about 30, 40, 50, or more parts of additives per 100 parts of adhesive components (e.g., in a first part, a second part, or both parts of a two-part adhesive) may be effectively utilized.

In some embodiments, the adhesive composition (in some embodiments, the structural adhesive composition) of the present disclosure includes a silane coupling agent. Examples of suitable silane coupling agents include those represented by formula L-[R⁴Si(Y)₃]ₖ. In this formula, L is an amino group (e.g., primary or secondary amino group), a mercapto group (i.e., HS-), or an epoxy group (i.e., 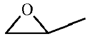 ). In some embodiments, L is an amino group or a mercapto group, which are capable of reacting with isocyanates. In formula L-[R⁴Si(Y)₃]ₖ, k is typically 1, but when L is an amino group, k is 1 or 2. In formula L-[R⁴Si(Y)₃]ₖ, R⁴ is alkylene (e.g., having up to 8, 6, or 4 carbon atoms) optionally interrupted by at least one ether linkage, and Y is a hydroylzable group such as halogen (i.e., fluoride, chloride, bromide, or iodide), alkoxy (i.e., -O-alkyl), acyloxy (i.e., -OC(O)alkyl), or aryloxy (i.e., -O-aryl). Silane coupling agents can be useful for promoting adhesion between the polyurethane and a filler (e.g., siliceous filler) in the adhesive composition or between the polyurethane and a substrate onto which it is dispensed. Examples of useful silane coupling agents include 3-glycidoxypropyltrimethoxysilane, available, for example, from Dow Coming Corporation, Midland, Michigan, under the trade designation "DOW CORNING Z-6040 SILANE"; bis(trimethoxysilylpropyl)amine available, for example, from Gelest, Morrisville, PA; (3-aminopropyl)trimethoxysilane, (3-aminopropyl)triethoxysilane), (3-mercaptopropyl)trimethoxysilane, and (3-mercaptopropyl)triethoxysilane available, for example, from Sigma-Aldrich, St. Louis, MO. Silane coupling agents can be present in the adhesive composition in an amount of up to 5, 4, 3, 2, or 1 weight percent, based on the total weight of the adhesive composition (e.g., in a first part, a second part, or both parts of a two-part adhesive composition). Coupling agents including amino groups can be incorporated into the second part of a two-part adhesive composition (including isocyanate-reactive compounds), for example.

In some embodiments, the adhesive composition (in some embodiments, the structural adhesive composition) of the present disclosure includes a plasticizer. Useful plasticizers can include nonfunctional plasticizers such as aliphatic and aromatic hydrocarbons, alkyl esters, alkyl ethers, aryl esters, and aryl ethers. Examples of plasticizers include straight and branched alkylphthalates, such as diisononyl phthalate, dioctyl phthalate and dibutyl phthalate, a partially hydrogenated terpene commercially available as "HB-40", trioctyl phosphate, alkylsulfonic acid esters of phenol, toluenesulfamide, adipic acid esters, castor oil, xylene, 1-methyl-2-pyrrolidinone and toluene. In some embodiments, plasticizers are branched plasticizers, such as branched chain alkyl phthalates for example di-isononyl phthalates (available under the trade designation PLATINOL N from BASF. The adhesive composition (in some embodiments, structural adhesive composition) may contain plasticizers in both components. The amount of plasticizer used may be an amount sufficient to give the desired rheological properties and disperse the components in the adhesive composition. In some embodiments, a plasticizer is present in about 1, 5, or 10 percent by weight or greater or about 50, 40, 10, 5, 4, 3, 2, or 1 weight percent, based on the total weight of the adhesive composition. For a two-part adhesive composition according to the present disclosure, the plasticizer can be added to first part, the second part, or both. In some embodiments, the plasticizer is added to the second part.

In use, the components of two-part compositions are mixed. For two-part compositions to be most easily used in commercial and industrial environments, the volume ratio at which the two parts are combined is preferably a convenient whole number. This facilitates application of the curable composition with conventional, commercially available dispensers including static and dynamic mixing. Such dispensers with static mixing are shown in U.S. Pat. Nos. 4,538,920 and 5,082,147 and are available from Conprotec, Inc. (Salem, N.J.) under the trade name MIXPAC of Medmix GmbH, Baar, Switzerland. Typically, these dispensers use a pair of tubular receptacles arranged side-by-side with each tube being intended to receive one of the two parts of the polymerizable composition. Two plungers, one for each tube, are simultaneously advanced (e.g., manually or by a hand-actuated ratcheting mechanism) to evacuate the contents of the tubes into a common, hollow, elongated mixing chamber that may also contain a static mixer to facilitate blending of the two parts. The blended polymerizable composition is extruded from the mixing chamber onto a substrate. When using electrically-driven equipment, dynamic mixing may be used. Once the tubes have been emptied, they can be replaced with fresh tubes and the application process continued. The volumetric ratio at which the two parts of the polymerizable composition are combined is controlled by the diameter of the tubes. (Each plunger is sized to be received within a tube of fixed diameter, and the plungers are advanced into the tubes at the same speed.) A single dispenser is often intended for use with a variety of different two-part polymerizable compositions and the plungers are sized to deliver the two parts of the polymerizable composition at a convenient mix ratio. Some common mix ratios are 1:1, 2:1, 4:1 and 10:1 and can also be odd ratios. In some embodiments, the two parts are blended at a mix ratio of about 1:1.

In some embodiments, the compound of the present disclosure is present in a range from 0.1 weight percent to 20 weight percent, based on the total weight of the adhesive composition (in some embodiments, the structural adhesive composition). In some embodiments, the compound of the present disclosure is present in a range from 0.1 weight percent to 10 weight percent, 0.1 weight percent to 5 weight percent, or 1 weight percent to 5 weight percent, based on the total weight of the adhesive composition (e.g., the first part, the second part, or both parts of a two-part adhesive).

Adhesive compositions of the present disclosure may be used, for example, to bond a first substrate to a second substrate to provide a bonded article. Many types of substrates may be bonded with compositions of the present disclosure such as metal (e.g., galvanized steel, stainless steel, or aluminum), glass (e.g., which may be coated with indium tin oxide), a polymer (e.g., a plastic, rubber, thermoplastic elastomer, or thermoset), or a composite. A composite material may be made from any two or more constituent materials with different physical or chemical properties. When the constituents are combined to make a composite, a material having characteristics different from the individual components is typically achieved. Some examples of useful composites include fiber-reinforced polymers (e.g., carbon fiber reinforced epoxies and glass-reinforced plastic), metal matrix compositions, and ceramic matrix composites. Useful polymeric substrates that can be bonded include polymers such as polyolefins (polypropylene, polyethylene, high density polyethylene, blends of polypropylene), fluoropolymers, polyamide 6 (PA6), polyamide 6,6, acrylonitrile butadiene styrene (ABS), polycarbonate (PC), PC/ABS blends, polyvinyl chloride (PVC), polyamide (PA), polyurethane (PUR), thermoplastic elastomers (TPE), polyolefin elastomers (e.g., ethylene-propylene rubber, ethylene-propylene-diene rubber), fluoropolymer elastomers, polyoxymethylene (POM), polystyrene, poly(methyl) methacrylate (PMMA), polyvinyl chloride (PVC), polyetheretherketone (PEEK), and combinations thereof. The substrate may also include a metal coating on such polymers.

The present disclosure provides an article bonded with the composition disclosed herein in any of its embodiments, wherein the composition is cured, and wherein the article comprises at least one of plastic, metal, or glass, in some embodiments, at least one of metal or glass.

The present disclosure provides a method of making a bonded article comprising a first substrate and a second substrate. The method includes applying the composition of present disclosure on at least one of the first substrate or the second substrate, contacting at least a portion of the composition with at least a portion of one surface of the second substrate, and allowing the composition to at least partially cure to make the bonded article. In some embodiments, the method includes combining the first part and the second part of the two-part composition to provide an adhesive composition, applying the adhesive composition to at least a portion of one surface of the first substrate, adhering the first substrate and the second substrate using the adhesive composition, and allowing the adhesive composition to at least partially cure to make the bonded article. In some embodiments, at least one of the first substrate or second substrate comprises at least one plastic. In some embodiments, at least one of the first substrate or second substrate comprises at least one of a metal or glass. The method can be carried out with or without subjecting the low-surface-energy plastic to flame treatment, corona discharge, plasma treatment, oxidation by ozone or oxidizing acids, sputter etching, primer treatment, or any combination thereof.

When the first part and the second part are combined, the composition is desirably used to bond the first substrate and the second substrate during the worklife of the composition, as described above. While it is not practical to enumerate a particular curing temperature suitable for all situations, generally suitable temperatures are in a range from about 23 °C to about 200 °C. In some embodiments, advantageously, the composition can be cured at room temperature (e.g., 23 °C to 30 °C, or below 40 °C), in at least 5 minutes, 10 minutes, 20 minutes, 30 minutes, or 45 minutes, for example. In some embodiments, advantageously, the composition can be cured at room temperature (e.g., 23 °C to 30 °C, or below 40 °C), within up to 60 minutes, 90 minutes, 120 minutes, 6 hours, 12 hours, 24 hours, 48 hours, or 72 hours for example. Full strength will normally be reached in less than 24 hours under ambient conditions. Post-curing at an elevated temperature may also be used if desired.

The first and second substrates can be joined together with pressure to force excess composition out of the bond line. This may be advantageous for displacing composition that may have advanced too far in cure. The typical bond line thickness is about 0.1 mm to 0.3 mm but may exceed 1.0 mm when gap filling is needed.

The composition of the present disclosure can be useful, for example, for bonding electronic articles and automotive (e.g., electronic vehicles) and aerospace components.

As shown in the Examples, below, the compound of the present disclosure generally improves the adhesion between metal (e.g., aluminum) substrates. Also, Examples 1 to 8 of the compound of the present disclosure each increase the overlap shear strength of the bond between aluminum and glass by at least about 50%, relative to the overlap shear strength observed for a control composition that does not include the compound. In some embodiments, the compound of the present disclosure increases the overlap shear strength of the bond between aluminum and glass by 100% or more.

### Some Embodiments of the Disclosure

In a first embodiment, the present disclosure provides a compound represented by formula: 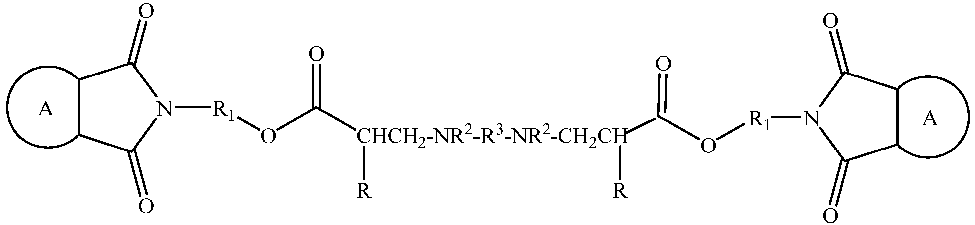 wherein
each A independently represents a saturated, unsaturated, or aromatic ring;
each R is independently hydrogen or methyl;
each R¹ is independently alkylene optionally interrupted by one or more -O- groups;
each R³ is independently alkylene, arylene, or alkylene optionally interrupted or terminated by arylene, optionally interrupted by one or more -O- groups or one to three -NR²- groups, and optionally terminated by -N(R²)₂; and
each R² is independently hydrogen, alkyl, aryl, arylalkylenyl, or 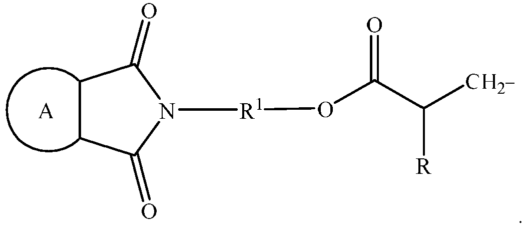 In a second embodiment, the present disclosure provides the compound of the first embodiment, wherein A is a saturated, six-membered ring, and wherein R¹ is linear alkylene having 2 to 4 carbon atoms. In a third embodiment, the present disclosure provides the compound of the first or second embodiment, wherein R³ is alkylene optionally interrupted by one or more -O- groups or one to three -NR²- groups, and optionally terminated by -N(R²)₂; and wherein each R² is independently hydrogen or 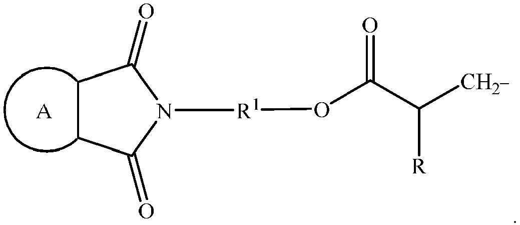 In a fourth embodiment, the present disclosure provides the compound of any one of the first to third embodiments, wherein R³ is alkylene optionally interrupted by one or more -O- groups. In a fifth embodiment, the present disclosure provides the composition of any one of the first to fourth embodiments, wherein each R² is hydrogen.

In a sixth embodiment, the present disclosure provides an adhesive composition comprising the compound of any one of the first to fifth embodiments. In a seventh embodiment, the present disclosure provides the adhesive composition of the sixth embodiment, wherein the adhesive composition is a structural adhesive composition. In an eighth embodiment, the present disclosure provides the adhesive composition of the sixth or seventh embodiments, wherein the adhesive composition is a two-part structural adhesive composition. In a ninth embodiment, the present disclosure provides the adhesive composition of any one of the sixth to eighth embodiments, wherein the adhesive composition is one part of a two-part structural adhesive composition. In a tenth embodiment, the present disclosure provides the adhesive composition of any one of the sixth to ninth embodiments, further comprising at least one polyol. In an eleventh embodiment, the present disclosure provides the adhesive composition of any one of the sixth to tenth embodiments, wherein the adhesive composition is a polyurethane adhesive composition. In a twelfth embodiment, the present disclosure provides the adhesive composition of any one of the fifth to eleventh embodiments, further comprising inorganic filler. In a thirteenth embodiment, the present disclosure provides the adhesive composition of any one of the fifth to twelfth embodiments, wherein the compound is present in a range from 0.1 weight percent to 20 weight percent, based on the total weight of the adhesive composition.

In a fourteenth embodiment, the present disclosure provides a method of bonding a first substrate and a second substrate, the method comprising applying the adhesive composition of any one of the sixth to thirteenth embodiments to at least a portion of one surface of the first substrate, contacting at least a portion of the adhesive composition with at least a portion of one surface of the second substrate; and allowing the adhesive composition to at least partially cure and form the structural adhesive. In a fifteenth embodiment, the present disclosure provides the method of any one of the fourteenth embodiment, wherein at least one of the first substrate or the second substrate comprises at least one of metal, glass, or plastic. **In** a sixteenth embodiment, the present disclosure provides the method of the fourteenth or fifteenth embodiment, wherein the adhesive composition is a first part of a two-part structural adhesive composition, the method further comprising combining the first part and a second part to provide a curable adhesive composition. **In** a seventeenth embodiment, the present disclosure provides bonded article comprising the structural adhesive bonded to the first substrate and the second substrate, the bonded article prepared according to the method of any one of the fourteenth to sixteenth embodiments.

**In** order that this disclosure can be more fully understood, the following examples are set forth. It should be understood that these examples are for illustrative purposes only and are not to be construed as limiting this disclosure in any manner.

### EXAMPLES

Unless otherwise noted, all parts, percentages, ratios, etc. in the Examples and the rest of the specification are by weight, and all reagents used in the examples were obtained, or are available, from general chemical suppliers such as, for example, Sigma-Aldrich, St. Louis, MO, or may be synthesized by conventional methods. The following abbreviations are used in this section: cm = centimeter, mm = millimeters, µm = micrometer, g = grams, lb = pound, mmole = milli-mole, °C = degrees Celsius, kN = kilo-Newton, and N = Newton. Unless otherwise noted, all parts, percentages, ratios, etc. in the Examples and the rest of the specification are by weight.

**Table 1: Materials List**

| Abbreviation | Description |
|---|---|
| Diamine 1 | Cycloaliphatic propoxylated diamine, obtained under the trade designation "JEFFAMINE RFD-270", obtained from Huntsman Corporation, The Woodlands, TX |
| Diamine 2 | Polypropylene glycol diamine, obtained under the trade designation "JEFFAMINE D-230", from Huntsman Corporation |
| Diamine 3 | Polypropylene glycol diamine, obtained under the trade designation "JEFFAMINE D-400", from Huntsman Corporation |
| Diamine 4 | Polypropylene glycol diamine, obtained under the trade designation "JEFFAMINE D-2000", from Huntsman Corporation |
| Triamine 5 | Polypropylene glycol Triamine, obtained under the trade designation "JEFFAMINE T-3000", from Huntsman Corporation |
| Diamine 6 | Meta-xylenediamine, obtained from Mitsubishi Chemical Company, Chiyoda City, Tokyo, Japan |
| Diamine 7 | 4,7,10-Trioxatridecane-1,13-Diamine, obtained from BASF, Ludwigshafen, Germany |
| Acrylate | 6H-dehydrophthalimidoethyl acrylate, obtained under the trade designation "MIRAMER M1088", from Miwon North America, Exton, PA |
| Polyol | Ring-opened epoxidized soybean oil-based polyol, obtained under the trade designation "BIOH 5000", from Cargill, Minneapolis, MN |
| Castor Oil | #1 Refined Castor Oil, obtained from Vertellus, Indianapolis, IN |
| DPG | Dipropylene glycol, obtained from Dow Chemical Co., Midland, MI |
| DABCO | 1,4-diazabicyclo[2.2.2]octane, obtained from Millipore Sigma, Burlington, MA |
| Amino Silane | Bipodal trimethoxysilane-amine, obtained under the trade designation "SILQUEST A-1170", obtained from Momentive Performance Materials, Niskayuna, NY |
| 5A MS | Molecular Sieves, size: 5Å obtained from Thermo Fisher Scientific, Waltham, MA |
| Fumed Silica | Hydrophobic fumed silica, obtained under the trade designation "CAB-O-SIL TS-610", from Cabot Corporation, Boston, MA |
| Rheology Modifier | Rheology additive based on phyllosilicate, obtained under the trade designation "BYK CLAYTONE APA", obtained from BYK Company, Wallingford, CT |
| Talc | Powdered talc filler, obtained under the trade designation "NICRON 604", from Imerys, Paris, France |
| Carbon Black | Carbon black powder, obtained under the trade designation "MONARCH 120", obtained from Cabot Corporation |
| Aluminum | 1"x4"x1/16" coupons, obtained from Joseph T. Ryerson & Son, Inc., Coon Rapids, MN |
| Glass | 1" x 4" x ¼" coupons of clear glass, obtained from Cat-I glass, Deerfield, IL |

### Test Methods

### Overlap Shear Test

Each sample formulation was separately loaded into the 2-part side of a 2:1 dual syringe cartridge dispenser, using the accelerator from 3M SCOTCH-WELD DP6310NS Composite Bonding Adhesive (3M Company, St. Paul, MN) in the 1-part side of the dispenser in each case. All bonds were prepared by dispensing the sample formulation and accelerator through a static mixing tip. The resulting adhesives were used to prepare samples for the Overlap Shear Test samples on grit-blasted aluminum, and IPA-wiped glass. Overlap shear samples were 2.54 x 10.16 x 0.16 cm aluminum, or clear glass coupons using nominally 0.45 mm spacer beads with a 1.27 cm overlap. The bond line was clamped with binder clips during cure and the clips were removed after 24 hours at 25°C. Testing was run on a 5000 lb (22 kN) load cell for overlap shear. The values were an average of three specimens.

### Examples 1 to 8 (EX1-EX8): Preparation of Multi-functional Cyclic Imides

A 30-gram vial containing a magnetic stir bar was charged with Diamine 1 (1.00 g, 3.7 mmol) and the Acrylate (1.84 g, 7.4 mmol). The vial was capped loosely with a rubber septum and the contents were heated with magnetic stirring to a temperature of 60°C via a silicone oil bath. After 6 hours at temperature, and the resultant adducted product was cooled to 25°C and stored for further use. All subsequent adhesion promoters were synthesized using this procedure, varying the molar amounts to yield bipodal (2:1 acrylate:amine), tripodal (3:1 acrylate:amine), or quadrapodal (4:1 acrylate:amine). Table 2 presents Examples PEX1-PEX8 and CEX9. The materials used and their quantities are listed in Table 2. The molar ratio refers to the molar ratio of acrylate to diamine.

**Table 2. Adhesion Promoter Examples 1-8 (PEX1-PEX8) and OLS results**

| EX | Diamine #, weight (g) | Acrylate (g) | Molar Ratio | OLS (Al/Al. psi) | OLS (Al/Clear Glass, psi) |
|---|---|---|---|---|---|
| EX1 | 1, 1.00 | 1.84 | 2:1 | 1331.0 | 1129.3 |
| EX2 | 1, 1.00 | 3.69 | 4:1 | 1573.7 | 1119.3 |
| EX3 | 2, 1.00 | 2.08 | 2:1 | 1389.7 | 874.0 |
| EX4 | 3, 1.00 | 1.16 | 2:1 | 1206.7 | 844.7 |
| EX5 | 4, 1.00 | 0.25 | 2:1 | 1537.0 | 1179.3 |
| EX6 | 7, 1.00 | 2.26 | 2:1 | 1279.0 | 1087.3 |
| EX7 | 5, 1.00 | 0.25 | 3:1 | 1424.7 | 811.3 |
| EX8 | 6, 1.00 | 3.66 | 2:1 | 1080.0 | 763.0 |
| CEX9 | Control (No Cyclic Imide) | | | 1154.7 | 514.0 |

### Adhesive Composition

Polyol (44.766 wt%), castor oil (20.0 wt%), DPG (4 wt%), Diamine 1 (3 wt%), DABCO (0.145 wt%), Amino Silane (0.5 wt%), 5A MS (0.95 wt%), Fumed Silica (0.95 wt%), Rheology Modifier (2.039 wt%), Talc (20.44 wt%), the Example Cyclic Imide (3 wt%), and Carbon Black (0.21 wt%) were combined in a speed mixer cup and mixed at 2000 rpm for 4 minutes. A homogeneous, thick paste was thus produced and used as described in the Overlap Shear Test protocol.

## Claims

1. A compound represented by formula: 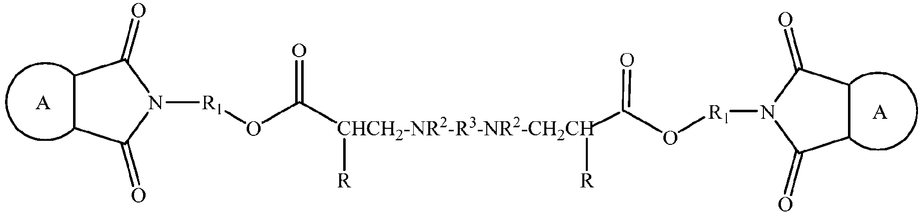 wherein
each A independently represents a saturated, unsaturated, or aromatic ring;
each R is independently hydrogen or methyl;
each R¹ is independently alkylene optionally interrupted by one or more -O- groups;
each R³ is independently alkylene, arylene, or alkylene optionally interrupted or terminated by arylene, optionally interrupted by one or more -O- groups or one to three -NR²- groups, and optionally terminated by -N(R²)₂; and
each R² is independently hydrogen, alkyl, aryl, arylalkylenyl, or

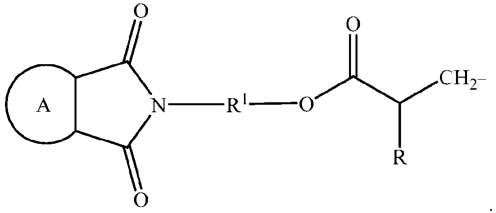

2. The compound of claim 1, wherein A is a saturated, six-membered ring, and wherein R¹ is linear alkylene having 2 to 4 carbon atoms.

3. The compound of claim 1 or 2, wherein R³ is alkylene optionally interrupted by one or more -O-groups or one to three -NR²- groups, and optionally terminated by -N(R²)₂; and wherein each R² is independently hydrogen or

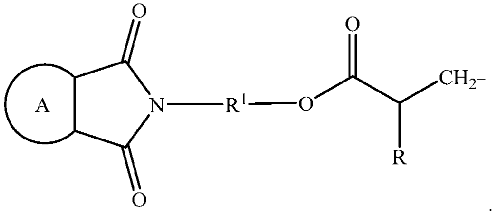

4. The compound of any one of claims 1 to 3, R³ is alkylene optionally interrupted by one or more -O- groups, and wherein each R² is hydrogen.

5. An adhesive composition comprising the compound of any one of claims 1 to 4.

6. The adhesive composition of claim 5, wherein the adhesive composition is a structural adhesive composition.

7. The adhesive composition of claim 5 or 6, wherein the adhesive composition is a two-part structural adhesive composition.

8. The adhesive composition of any one of claims 5 to 7, wherein the adhesive composition is one part of a two-part structural adhesive composition.

9. The adhesive composition of claim 8, further comprising at least one polyol.

10. The adhesive composition of any one of claims 6 to 9, wherein the adhesive composition is a polyurethane adhesive composition.

11. The adhesive composition of any one of claims 5 to 10, further comprising inorganic filler.

12. The adhesive composition of any one of claims 5 to 11, wherein the compound is present in a range from 0.1 weight percent to 20 weight percent. based on the total weight of the adhesive composition.

13. A method of bonding a first substrate and a second substrate, the method comprising:
applying the adhesive composition of any one of claims 6 to 12 to at least a portion of one surface of the first substrate;
contacting at least a portion of the adhesive composition with at least a portion of one surface of the second substrate; and
allowing the adhesive composition to at least partially cure and form the structural adhesive.

14. The method of claim 13, wherein at least one of the first substrate or the second substrate comprises at least one of metal, glass, or plastic.

15. The method of claim 13 or 14, wherein the adhesive composition is a first part of a two-part structural adhesive composition, the method further comprising combining the first part and a second part to provide a curable adhesive composition.

## Patentansprüche

1. Eine Verbindung, dargestellt durch die Formel: 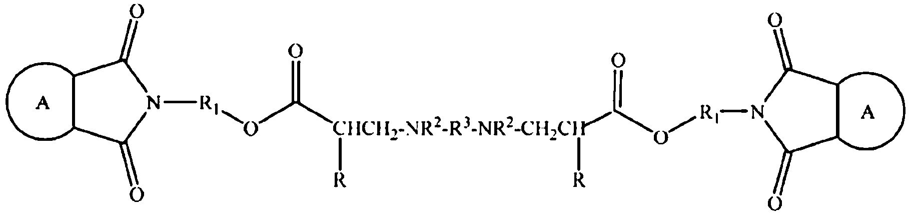 wobei
jedes A unabhängig einen gesättigten, ungesättigten oder aromatischen Ring darstellt;
jedes R unabhängig Wasserstoff oder Methyl ist;
jedes R¹ unabhängig Alkylen ist, das gegebenenfalls durch eine oder mehrere -O-Gruppen unterbrochen ist;
jedes R³ unabhängig Alkylen, Arylen oder Alkylen ist, das gegebenenfalls durch Arylen unterbrochen oder terminiert ist, gegebenenfalls durch eine oder mehrere -O-Gruppen oder eine bis drei -NR²-Gruppen unterbrochen ist und gegebenenfalls durch -N(R²)₂ terminiert ist; und
jedes R² unabhängig Wasserstoff, Alkyl, Aryl, Arylalkylenyl ist, oder

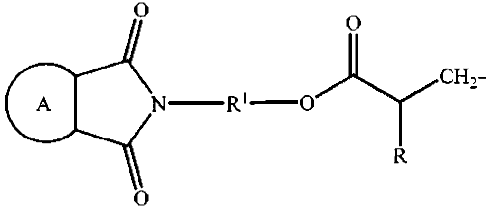

2. Die Verbindung nach Anspruch 1, wobei A ein gesättigter, sechsgliedriger Ring ist und wobei R¹ lineares Alkylen ist, das 2 bis 4 Kohlenstoffatome aufweist.

3. Die Verbindung nach Anspruch 1 oder 2, wobei R³ Alkylen ist, das gegebenenfalls durch eine oder mehrere -O-Gruppen oder eine bis drei -NR²-Gruppen unterbrochen ist und gegebenenfalls durch -N(R²)₂ terminiert ist; und wobei jedes R² unabhängig Wasserstoff ist oder

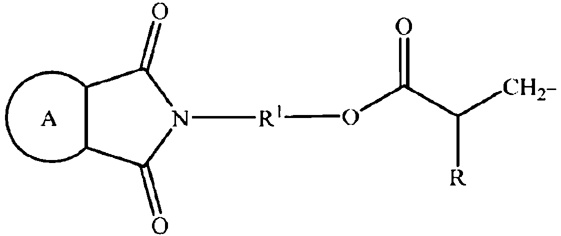

4. Die Verbindung nach einem der Ansprüche 1 bis 3, wobei R³ Alkylen ist, das gegebenenfalls durch eine oder mehrere -O-Gruppen unterbrochen ist und wobei jedes R² Wasserstoff ist.

5. Eine Klebstoffzusammensetzung, umfassend die Verbindung nach einem der Ansprüche 1 bis 4.

6. Die Klebstoffzusammensetzung nach Anspruch 5, wobei die Klebstoffzusammensetzung eine Strukturklebstoffzusammensetzung ist.

7. Die Klebstoffzusammensetzung nach Anspruch 5 oder 6, wobei die Klebstoffzusammensetzung eine zweiteilige Strukturklebstoffzusammensetzung ist.

8. Die Klebstoffzusammensetzung nach einem der Ansprüche 5 bis 7, wobei die Klebstoffzusammensetzung ein Teil einer zweiteiligen Strukturklebstoffzusammensetzung ist.

9. Die Klebstoffzusammensetzung nach Anspruch 8, ferner umfassend mindestens ein Polyol.

10. Die Klebstoffzusammensetzung nach einem der Ansprüche 6 bis 9, wobei die Klebstoffzusammensetzung eine Polyurethan-Klebstoffzusammensetzung ist.

11. Die Klebstoffzusammensetzung nach einem der Ansprüche 5 bis 10, ferner umfassend einen anorganischen Füllstoff.

12. Die Klebstoffzusammensetzung nach einem der Ansprüche 5 bis 11, wobei die Verbindung in einem Bereich von 0,1 Gewichtsprozent bis 20 Gewichtsprozent, bezogen auf das Gesamtvolumen der Klebstoffzusammensetzung, vorliegt.

13. Ein Verfahren zum Binden eines ersten Substrats und eines zweiten Substrats, das Verfahren umfassend:
Aufbringen der Klebstoffzusammensetzung nach einem der Ansprüche 6 bis 12 auf mindestens einen Abschnitt einer Oberfläche des ersten Substrats;
Inberührungbringen mindestens eines Abschnitts der Klebstoffzusammensetzung mit mindestens einem Abschnitt einer Oberfläche des zweiten Substrats; und
Ermöglichen, dass die Klebstoffzusammensetzung mindestens teilweise aushärtet und den Strukturklebstoff ausbildet.

14. Das Verfahren nach Anspruch 13, wobei mindestens eines von dem ersten Substrat oder dem zweiten Substrat mindestens eines von Metall, Glas oder Kunststoff umfasst.

15. Das Verfahren nach Anspruch 13 oder 14, wobei die Klebstoffzusammensetzung ein erster Teil einer zweiteiligen Strukturklebstoffzusammensetzung ist, das Verfahren ferner umfassend ein Kombinieren des ersten Teils und eines zweiten Teils, um eine härtbare Klebstoffzusammensetzung bereitzustellen.

## Revendications

1. Composé représenté par la formule : 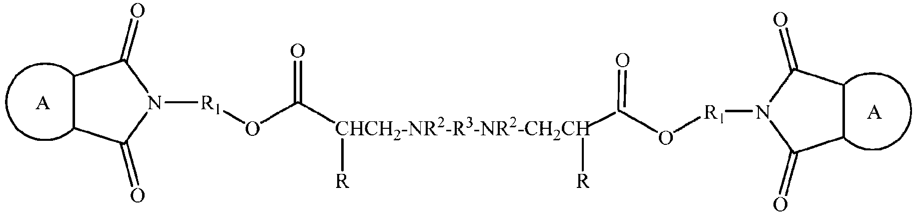 dans lequel
chaque A représente indépendamment un cycle saturé, insaturé, ou aromatique ;
chaque R est indépendamment hydrogène ou méthyle ;
chaque R¹ est indépendamment alkylène éventuellement interrompu par un ou plusieurs groupes -O- ;
chaque R³ est indépendamment alkylène, arylène, ou alkylène éventuellement interrompu ou terminé par arylène, éventuellement interrompu par un ou plusieurs groupes -O- ou un à trois groupes -NR²-, et éventuellement terminé par -N(R²)₂ ; et
chaque R² est indépendamment hydrogène, alkyle, aryle, arylalkylenyle, ou

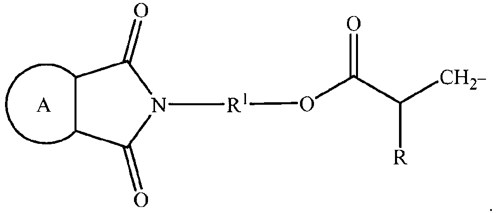

2. Composé selon la revendication 1, dans lequel A est un cycle saturé à six chaînons, et dans lequel R¹ est un alkylène linéaire ayant 2 à 4 atomes de carbone.

3. Composé selon la revendication 1 ou 2, dans lequel R³ est alkylène éventuellement interrompu par un ou plusieurs groupes -O- ou un à trois groupes -NR²-, et éventuellement terminé par -N(R²)₂ ; et dans lequel chaque R² est 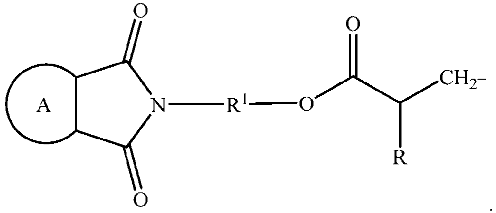 indépendamment hydrogène ou

4. Composé selon l'une quelconque des revendications 1 à 3, R³ est alkylène éventuellement interrompu par un ou plusieurs groupes -O-, et dans lequel chaque R² est hydrogène.

5. Composition adhésive comprenant le composé selon l'une quelconque des revendications 1 à 4.

6. Composition adhésive selon la revendication 5, dans laquelle la composition adhésive est une composition adhésive structurelle.

7. Composition adhésive selon la revendication 5 ou 6, dans laquelle la composition adhésive est une composition adhésive structurelle.

8. Composition adhésive selon l'une quelconque des revendications 5 à 7, dans laquelle la composition adhésive est une partie d'une composition adhésive structurelle en deux parties.

9. Composition adhésive selon la revendication 8, comprenant en outre au moins un polyol.

10. Composition adhésive selon l'une quelconque des revendications 6 à 9, dans laquelle la composition adhésive est une composition adhésive de polyuréthane.

11. Composition adhésive selon l'une quelconque des revendications 5 à 10, comprenant en outre une charge inorganique.

12. Composition adhésive selon l'une quelconque des revendications 5 à 11, dans laquelle le composé est présent dans une plage de 0,1 pour cent en poids à 20 pour cent en poids sur la base du poids total de la composition adhésive.

13. Procédé de collage d'un premier substrat et d'un second substrat, le procédé comprenant :
l'application de la composition adhésive selon l'une quelconque des revendications 6 à 12 sur au moins une partie d'une surface du premier substrat ;
la mise en contact d'au moins une partie de la composition adhésive avec au moins une partie d'une surface du second substrat ; et
laisser la composition adhésive durcir au moins partiellement et former l'adhésif structurel.

14. Procédé selon la revendication 13, dans lequel au moins l'un parmi le premier substrat ou le second substrat comprend au moins l'un parmi du métal, verre ou plastique.

15. Procédé selon la revendication 13 ou 14, dans lequel la composition adhésive est une première partie d'une composition adhésive structurelle bicomposante, le procédé comprenant en outre la combinaison de la première partie et d'une seconde partie pour obtenir une composition adhésive durcissable.
